# EUROPÄISCHE PATENTANMELDUNG

(11) **EP 0 556 647 A1**
(43) Veröffentlichungstag der Anmeldung: **25.08.1993**
(21) Anmeldenummer: 93101695.0
(22) Anmeldetag: 04.02.1993
(51) Int. Cl.: C07D 237/14, C07D 237/16, A01N 43/58

(54) **Herbizide N-Cyanopyridazinone**

(30) Priorität: 17.02.1992 AT 258/92
(71) Anmelder: SANDOZ LTD., CH-4002 Basel (CH); SANDOZ-ERFINDUNGEN Verwaltungsgesellschaft m.b.H., A-1230 Vienna (AT); SANDOZ-PATENT-GMBH, D-79539 Lörrach (DE)
(72) Erfinder: Leitner, Harald, Dr., A-4064 Oftering (AT); Wörther, Rudolf Helmut, Dr., A-4020 Linz (AT); Korntner, Horst, A-4020 Linz (AT); Schneider, Rudolf, Dipl.-Ing. Dr., A-4040 Linz (AT); Auer, Engelbert, A-4060 Leonding (AT); Kores, Dietmar, Dr., A-4060 Leonding (AT); Tramberger, Hermann, A-3353 Seitenstetten (AT)

(57) **Zusammenfassung**

Neue herbizide N-Cyanopyridazinone der allgemeinen Formel 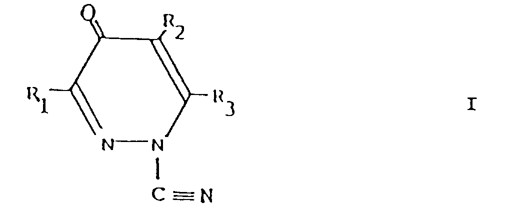
in der die Reste R₁, R₂ und R₃ unabhängig voneinander Wasserstoff, Halogen-, Amino-, Nitro-, Cyano-, Alkyl-, Aryl-, Aralkyl-, Alkoxy- oder Aryloxygruppen bedeuten, ein Verfahren zu deren Herstellung, ein herbizides Mittel, das diese enthält und ein Verfahren zur Bekämpfung von Unkräutern.

## Beschreibung

Die Erfindung betrifft neue N-Cyanopyridazinone mit herbizider Wirkung, ein Verfahren zu deren Herstellung, herbizide Mittel, die die neuen N-Cyanopyridazinone enthalten, ein Verfahren zu deren Herstellung und ein Verfahren zur Bekämpfung von Unkräutern.

In DE-OS 26 28 990 sind herbizide 3,5-Diphenyl-4(1H)-pyridazinone und -pyridazinthione, die am 1-Stickstoff durch Alkylgruppen mit 1 bis 3 C-Atomen substituiert sind, geoffenbart. Die für eine herbizide Wirkung nötige Aufwandmenge ist aber exorbitant hoch.

Es wurden nun unerwarteterweise neue N-Cyanopyridazinone-4, die bereits in geringer Aufwandmenge eine ausgezeichnete herbizide Wirkung aufweisen, gefunden.

Gegenstand der Erfindung sind daher N-Cyanopyridazinone der allgemeinen Formel 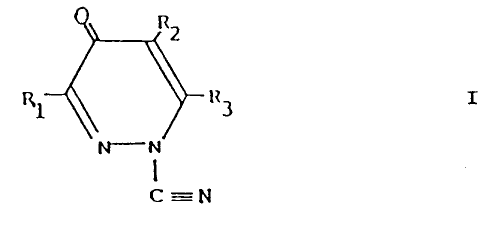
in der R₁, R₂ und R₃ unabhängig voneinander Wasserstoff, Halogen-, Amino-, Nitro-, Cyano-, Alkyl-, Aryl-, Aralkyl-, Alkoxy- oder Aryloxygruppen bedeuten.

Unter Halogengruppe ist dabei Fluor, Chlor, Brom oder Jod, bevorzugt Chlor oder Brom, unter Aminogruppen sind unsubstituierte oder duch Alkyl- oder Arylgruppen substituierte Aminogruppen oder Aminogruppen in oder an einem heterocyclischen Ring zu verstehen. Alkylgruppen sind unsubstituierte oder durch Halogen-, Amino-, Nitro-, Cyano-, Alkoxy- oder Aryloxygruppen substituierte, bevorzugt unsubstituierte oder durch Halogen substituierte Alkylgruppen, die ganz bevorzugt 1 bis 4 C-Atome aufweisen, beispielsweise Methyl-, Äthyl-, iso-Propyl-, Butyl-, Trifluormethyl-, Trifluoräthylgruppen. Unter Arylgruppen sind unsubstitituerte oder durch Halogen-, Amino-, Nitro-, Cyano-, Alkyl-, Aryl-, Aralkyl-, Alkoxy- oder Aryloxygruppen ein oder mehrfach substituierte Phenylgruppen, bevorzugt unsubstituierte oder durch Halogen-, Nitro-, Cyano- oder Alkylgruppen ein- oder mehrfach substituierte Phenylgruppen zu verstehen.
In den Alkoxy- oder Aryloxygruppen haben die Alkyl- oder Arylgruppen die oben angeführte Bedeutung. Bevorzugt sind dabei Alkoxygruppen, besonders bevorzugt solche mit 1 bis 8, ganz besonders bevorzugt solche mit 1 bis 6 C-Atomen. Bevorzugt sind Verbindungen der allgemeinen Formel I, in denen die Reste R₁, R₂ und R₃ unabhängig voneinander Wasserstoff, Halogen-, Alkoxy- oder Arylgruppen bedeuten.
Besonders bevorzugt bedeuten R₁ eine Halogen- oder eine Arylgruppe, ganz besonders bevorzugt eine unsubstituierte oder ein- oder mehrfach durch Halogen-, Nitro-, Cyano-, Alkyl- oder Alkoxygruppen substituierte Phenylgruppe, R₂ Wasserstoff oder eine Halogengruppe und R₃ eine Alkoxy-, eine Halogen- oder eine Arylgruppe, die besonders bevorzugt eine unsubstituierte oder durch Halogen substituierte Phenylgruppe ist.

Gegenstand der Erfindung ist auch ein Verfahren zur Herstellung von N-Cyanopyridazinonen der allgemeinen Formel 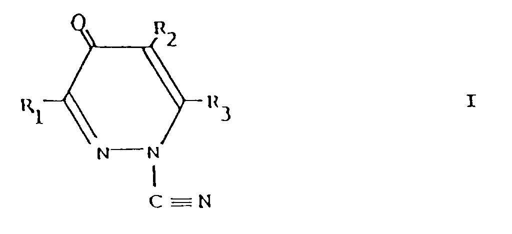
in der R₁, R₂ und R₃ unabhängig voneinander Wasserstoff, Halogen-, Amino-, Nitro-, Cyano-, Alkyl-, Aryl-, Aralkyl-, Alkoxy- oder Aryloxygruppen bedeuten, das dadurch gekennzeichnet ist, daß ein Hydroxypyridazin der allgemeinen Formel 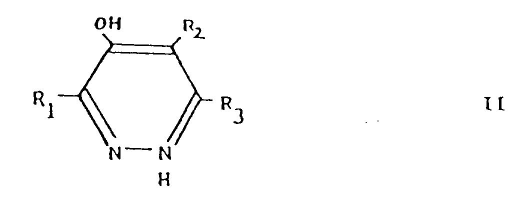
in der R₁, R₂ und R₃ die obgenannte Bedeutung haben, in einem unter den Reaktionsbedingungen inerten Verdünnungsmittel gelöst oder suspendiert, mit einer Base und mit einem Halogencyan versetzt und reagieren gelassen wird, worauf das gebildete Cyanopyridazinon der allgemeinen Formel I, aus dem Reaktionsgemisch isoliert wird.

Zur Herstellung der Verbindungen der allgemeinen Formel I kann das Hydroxypyridazin der allgemeinen Formel II in einem unter den Reaktionsbedingungen inerten Verdünnungsmittel, beispielsweise chlorierte Kohlenwasserstoffe wie Methylenchlorid, Äthylenchlorid, Trichloräthylen, Ketone wie Aceton, Dibutylketon, Äther wie Diisopropyläther, Dioxan, Säureamide wie Dimethylformamid, Dialkylsulfoxide wie Dimethylsulfoxid, Alkohole wie Methanol, Äthanol, Diisopropylalkohol, Wasser oder Mischungen der angeführten Verdünnungsmittel gelöst oder suspendiert mit einer anorganischen oder organischen Base, beispielsweise Natrium- oder Kaliumhydroxid, -hydrid, -carbonat, Ammoniak, Amine wie Triäthylamin, Pyridin, bevorzugt mit Aminen, besonders bevorzugt mit Triäthylamin und mit einem Halogencyan wie Chlor-, Brom- oder Jodcyan, bevorzugt Chlor- oder Bromcyan reagieren gelassen werden. Dabei ist es ungewöhnlich, daS die Reaktion auch in Wasser oder wäßrigen Mischungen organischer Verdünnungsmittel abläuft.

Das Halogencyan und die Base werden im allgemeinen mindestens äguimolar oder in einem Überschuß bezogen auf das Hydroxypyridazin der Formel II eingesetzt, wobei es aber in manchen Fällen auch von Vorteil sein kann, das Hydroxypyridazin der Formel II im Überschuß einzusetzen.
Die Zugabe der Base und des Halogencyans erfolgt, abhängig von der chemischen Struktur der verwendeten Reaktionspartner, bei Temperaturen von etwa -70 °C bis zum Siedepunkt des verwendeten Verdünnungsmittels. Bevorzugt werden die Base und das Halogencyan bei Temperaturen von -10 °C bis 60 °C, ganz bevorzugt von etwa 0 °C bis Raumtemperatur zugegeben. Die Temperatur der Reaktionsmischung steigt bei der Zugabe des Halogencyans im allgemeinen an. Nach beendeter Zugabe, die gegebenenfalls unter Kühlung erfolgen kann, kann bei gegebener Temperatur weiterreagieren gelassen werden, oder die Reaktionsmischung wird zur Vervollständigung der Reaktion, gegebenenfalls bis zum Siedepunkt des verwendeten Verdünnungsmittels, erhitzt. Im allgemeinen ist ein Erhitzen jedoch nicht notwendig. Bevorzugt werden die Base und das Halogencyan bei Raumtemperatur zugegeben und bei der dabei erreichten Temperatur ohne von außen zu kühlen oder zu erhitzen reagieren gelassen.

Dabei reagiert das Halogencyan im Gegensatz zu Acylhalogeniden nicht am Sauerstoffatom in Position 4, sondern unerwarteterweise am Stickstoffatom in Position 1 des Pyridazinringes.

Die Umsetzung kann auf übliche Art und Weise, beispielsweise chromatographisch verfolgt werden. Nach beendeter Reaktion wird das, aus dem Halogen des Halogencyans mit der Base gebildete Salz aus dem Reaktionsgemisch wie üblich, etwa durch Abfiltrieren, Extraktion, entfernt, worauf das organische Verdünnungsmittel abgedampft wird. Dabei verbleibt das gewünschte N-Cyanopyridazinon der allgemeinen Formel I als öliger oder kristalliner Rückstand. Falls gewünscht, kann der Rückstand noch einer weiteren Reinigungsoperation, z.B. Chromatographieren, Umkristallisieren, unterzogen werden.

Es hat sich herausgestellt, daß bei Verbindungen der Formel I, in denen R₁ eine Arylgruppe und R₃ eine Halogengruppe bedeutet, die Position 6, in der R₃ gebunden ist, besonders reaktionsfähig ist, sodaß R₃ durch andere funktionelle Gruppen ersetzt werden kann. So kann beispielsweise das Chloratom in der Verbindung 1-Cyano-3-phenyl-6-chlorpyridazinon-4 durch Umsetzung der Verbindung mit Natriumjodid in einem organischen Lösungsmittel in die Verbindung 1-Cyano-3-phenyl-6-jodpyridazinon-4 übergeführt werden. Es ist also auch möglich, Verbindungen der Formel I aus Verbindungen der Formel I durch Ersatz einer funktionellen Gruppe in der Postion 6 des Pyridazinonringes durch eine andere funktionelle Gruppe zu erhalten.

Die Herstellung der Ausgangsverbindungen der allgemeinen Formel II ist beispielsweise aus DE 40 13 734 bekannt und kann etwa durch Umsetzung entsprechender Pyridazine mit einem Alkalihydroxid oder durch Dealkylierung eines 4-Alkoxypyridazins, beispielsweise mit Hilfe von Alkalihydrid und Alkylthiol in einem unter den Reaktionsbedingungen inerten Verdünnungsmittel erfolgen.
So können etwa 3-Aryl-4-hydroxy-6-brompyridazin durch Umsetzung von 3-Aryl-4,6-dibrompyridazin mit Natriumhydroxid in Dioxan/Wasser und 3-Phenyl-4-hydroxy-6-methoxy-pyridazin aus 4,6-Dimethoxy-3-phenylpyridazin durch Umsetzung mit Natriumhydrid und Butanthiol in Dimethylformamid hergestellt werden. 3-Aryl-alkoxypyridazine sind beispielsweise aus den entsprechenden 3-Aryl-halogenpyridazinen durch Umsetzung mit Alkalialkoholat herstellbar. 3-Aryl-halogenpyridazine, wie etwa 3-(3'-Trifluormethylphenyl)-4,6-dichlorpyridazin können beispielsweise aus 3-Aryl-pyridazinon-6 durch Halogenierung in Phosphoroxidchlorid mit rotem Phosphor und elementarem Halogen hergestellt werden. Bei längerer Halogenierung können schrittweise alle Positionen 4, 5 und 6 des Pyridazinonringes halogeniert werden.

Die erfindungsgemäßen N-Cyanopyridazinone zeigen ausgezeichnete herbizide Wirkung und stellen somit eine Bereicherung der Technik dar. Sie eignen sich zur Bekämpfung von dikotylen, aber auch monokotylen Samenunkräutern in Kulturen wie z.B. Getreide, Mais, Erdnuß, Kohlgewächsen, Kichererbsen, Tomaten, Zwiebeln.

Besonders geeignet sind sie beispielsweise zur Bekämpfung von
Amaranthus retroflexus (AMARE) - zurückgekrümmter Amarant
Anthemis arvensis (ANTAR) - Acker-Hundskamille
Centaurea cyanus (CENCY) - Kornblume
Chenopodium album (CHEAL) - Weißer Gänsefuß
Echinochloa crus-galli (ECHCG) - Hühnerhirse
Galium aparine (GALAP) - Klettenlabkraut
Lapsana communis (LAPCO) - Rainkohl
Stellaria media (STEME) - Vogelmiere
in den oben genannten Kulturen.

Gegenstand der Erfindung ist auch ein herbizides Mittel, das dadurch gekennzeichnet ist, daß es mindestens ein N-Cyanopy-ridazinon der allgemeinen Formel I nach Anspruch 1 neben Hilfs- und/oder Trägerstoffen enthält.

Das herbizide Mittel wird in bekannter Weise formuliert, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von Tensiden, also Emulgatoren und/oder Dispergier- und/oder Netzmitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Dichlormethan, Trichlorbenzole, Aliphaten wie Cyclohexan oder Paraffine, z.B. Erdölfraktionen, Alkohole, wie Butanol oder Glykol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe, sowie Butan, Propan, Stickstoff und Kohlendioxid; als feste Trägerstoffe kommen in Frage: z.B. natürliche Gesteinsmehle, wie Kaoline, Tonerde, Talkum, Kreide, Quarz, Attapulgit, Montmorillonit oder Diatomeenerde und synthetische Produkte wie hochdisperse Kieselsäure, Aluminiumoxid und Silikate; als feste Trägestoffe für Granulate kommen in Frage: z.B. gebrochene und fraktionierte natürliche Gesteine, wie Calcit, Marmor, Bims, Sepiolith, Dolomit sowie synthetische Granulate aus anorganischen und organischen Mehlen, sowie Granulate aus organischem Material, wie Sägemehl, Kokosnuß-Schalen, Maiskolben und Tabakstengel; als Emulgiermittel und/oder schaumerzeugenden Mittel kommen in Frage: z.B. nichtionogene und ionogene Tenside, wie Polyoxyethylen-Sorbitan-Tallölester, Na-Oleylmethyltaurid, Polyoxyethylen-Fettsäure-Ester, Polyoxyethylen-Fettalkoholether, z.B. Alkylarylpolyglykolether, Alkylsulfonate, Alkylsulfate, Arylsulfate und Arylalkylsulfonate sowie Eiweißhydrolysate. Als Netzmittel können etwa polyoxyethylierte Alkylphenole, polyoxethylierte Oleyl- oder Stearylamine, Alkyl- oder Alkylphenylsulfonate eingesetzt werden. Als Dispergiermittel kommen in Frage: z.B. Ligninsulfonate oder Kondensationsprodukte von Arylsulfonaten mit Formaldehyd.

Es können in den Formulierungen Haft- und Verdickungsmittel wie Carboxymethylcellulose, Methylcellulose, natürliche und synthetische pulvrige, körnige und latexförmige Polymere verwendet werden, wie Gummi arabicum, Polyvinylalkohol, Polyvinylacetat.

Es können Farbstoffe, wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90 Gew.%.

Die Wirkstoffe können als solche, in Form ihrer Formulierungen oder aus daraus durch weiteres Verdünnen bereiteten Anwendungsformen, wie gebrauchsfertige Lösungen, Emulsionen, Suspensionen, Pulver, Pasten und Granulate angewendet werden. Benetzbare Pulver sind im Wasser gleichmäßig dispergierbare Präparate, die neben den Wirkstoffen außer gegebenenfalls Verdünnungs- bzw. Inertstoffen noch Netzmittel enthalten können. Emulgierbare Konzentrate können beispielsweise durch Auflösen der Wirkstoffe in einem organischen Lösungsmittel unter Zusatz von einem oder mehreren Emulgiermitteln hergestellt werden. Stäubemittel werden durch Vermahlen der Wirkstoffe mit fein verteilten festen Trägerstoffen erhalten.
Die Anwendung geschieht in üblicher Weise, z.B. durch Gießen, Tauchen, Spritzen, Sprühen, Vernebeln, Verdampfen, Injizieren, Verschlämmen, Stäuben, Streuen, Trockenbeizen, Feuchtbeizen, Nassbeizen, Schlämmbeizen oder Inkrustieren.

Mit den äußeren Bedingungen, wie Temperatur, Feuchtigkeit u.a. variiert die erforderliche Aufwandmenge an den erfindungsgemäßen Mitteln. Sie kann sich innerhalb weiter Grenzen bewegen und beträgt im allgemeinen zwischen 0,1 und 5 kg/ha Wirkstoffe, vorzugsweise 0,1 bis 1,7 kg/ha.

### Beispiel 1

10,35 g 3-Phenyl-4-hydroxy-6-chlor-pyridazin (0,05 Mol) wurden in 100 ml Aceton suspendiert und bei Raumtemperatur unter Rühren mit 6,07 g Triethylamin (0,06 Mol) versetzt. Nach etwa einer halben Stunde Rührens wurden der Reaktionsmischung 6,36 g Bromcyan (0,06 Mol), gelöst in Aceton, zugetropft, wobei die Temperatur der Reaktionsmischung leicht anstieg. Nach Rühren über Nacht wurde das abgeschiedene Triethylammoniumbromid abfiltriert und das organische Lösungsmittel abgedampft. Der kristallisierende Rückstand wurde in 100 ml Wasser digeriert, abfiltriert, mit Wasser gewaschen und getrocknet.

Dabei wurden 11,3 g 1-Cyano-3-phenyl-6-chlor-pyridazinon-4, das sind 97 % der Theorie, erhalten.
Nach dem Umkristallisieren aus Diisopropylether/Aceton = 7 : 3 wurden weiße Kristalle mit einem Schmelzpunkt von 120 bis 121 °C erhalten.

Ein ähnliches Ergebnis wurde bei Verwendung von 5,3 g Bromycan (0,05 Mol) unter sonst gleichen Bedingungen erhalten.

### Beispiel 2

12,55 g 3-Phenyl-4-hydroxy-6-brom-pyridazin (0,05 Mol) wurden in 100 ml Aceton suspendiert und bei Raumtemperatur unter Rühren mit 7,6 g Triethylamin (0,075 Mol) versetzt. Nach einer halben Stunde Rührens wurden 10,6 g Bromycan (0,1 Mol), gelöst in 100 ml Aceton, zugetropft, wobei die Temperatur leicht anstieg. Nach Rühren über Nacht wurde vom ausgefallenen Triethylammoniumbromid abfiltiert und das organische Lösungsmittel abgedampft. Der Rückstand wurde in 100 ml Wasser digeriert, der dabei erhaltene Feststoff abfiltiert, mit Wasser gewaschen und getrocknet.

Dabei wurden 12,6 g 1-Cyano-3-phenyl-6-brom-pyridazinon-4, das sind 91 % der Theorie, erhalten.

Zur weiteren Reinigung wurde über eine Kieselsäule mit Chloroform als Eluationsmittel chromatographiert und anschließend aus einem Aceton/Diisopropyläthergemisch umkristallisiert, wobei weisse Kristalle mit einem Schmelzpunkt von 152 bis 155 °C erhalten wurden.

### Beispiel 3

In einer Lösung von 80 g Natriumhydroxid in 1,6 l Wasser wurden 413,3 g 3-Phenyl-4-hydroxy-6-chlorpyridazin (2,0Mol) gelöst und mit 1,6 l Aceton versetzt. Dieser Lösung wurde bei 25°C unter Rühren 211,9 g Bromcyan (2,0Mol) zugegeben. Dabei stieg die Temperatur auf 31°C und der pH Wert sank von 9 auf 6. Nach 2 Stunden Rühren bei Raumtemperatur wurde der ausgefallene Niederschlag abgesaugt, mit wäßrigem Aceton gewaschen und getrocknet.
Dabei wurden 425 g 1-Cyano-3-phenyl-6-chlorpyridazinon-4 in einer Ausbeute von 92% der Theorie erhalten. Nach Rühren des Produktes in einer 5%igen, wäßrigen NaHCO₃ Lösung, Waschen mit Wasser und Trocknen wurde ein Schmelzpunkt von 117-120°C gemessen.

### Beispiel 4

In eine Lösung von 10,8 g Natriumchlorid und 1,7 ml konzentrierter Salzsäure in 577 ml Wasser wurden bei -3 bis 0°C 56,7 g Chlorgas eingeleitet und 217,7 ml einer 30 Gew%igen Lösung von Natriumcyanid in Wasser eingebracht, wobei eine wäßrige Lösung von Chlorcyan erhalten wurde.
Dieser Lösung wurden bei -5°C eine Lösung von 82,6 g 3-Phenyl-4-hydroxy-6-chlorpyridazin (0,4 Mol) und 16 g Natriumhydroxid in 417 ml Wasser und 667 ml Aceton zugegeben. Nach 4 Stunden Rühren bei Raumtemperatur wurde der gebildete Niederschlag abgesaugt, mit Wasser gewaschen und getrocknet.
Dabei wurden 90 g 1-Cyano-3-phenyl-6-chlorpyridazinon-4, das sind 97% der Theorie mit einem Schmelzpunkt von 117 bis 119°C erhalten.

### Beispiel 5

Wurde wie Beispiel 4, aber unter Verwendung von Dioxan/Wasser anstatt Aceton/Wasser durchgeführt. Dabei wurden 96 % der Theorie 1-Cyano-3-phenyl-6-chlorpyridazinon-4 mit einem Schmelzpunkt von 120 bis 121 °C erhalten.

### Beispiel 6

Wurde wie Beispiel 4, aber unter Verwendung von Wasser ohne Zusatz von Aceton als Lösungsmittel durchgeführt. Dabei wurden 97 % der Theorie 1-Cyano-3-phenyl-6-chlorpyridazinon-4 mit einem Schmelzpunkt von 117 bis 119 °C erhalten.

Auf die in den Beispielen 1 bis 6 beschriebene Art und Weise, unter Verwendung entsprechender Ausgangsstoffe, wurden die in Tabelle 1 angeführten Verbindungen erhalten.

**Tabelle 1**

| | **R₁** | **R₂** | **R₃** | **Fp (°C)** |
|---|---|---|---|---|
| **7** | Cl | H | Cl | 167-169 |
| **8** | Phenyl | H | OCH₃ | 185-190 |
| **9** | Phenyl | H | OC₄H₉ | 162-164 |
| **10** | Phenyl | H | Cl | 145-146 |
| **11** | 4-Br-Phenyl | H | Cl | 122-123 |
| **12** | 2-F-Phenyl | H | Cl | 157-159 |
| **13** | 3Cl,4Cl-Phenyl | H | Cl | 153-157 |
| **14** | 2Cl,3Cl,4Cl-Phenyl | H | Cl | 180-184 |
| **15** | 4-CN-Phenyl | H | Cl | 204-208 |
| **16** | 4-NO₂-Phenyl | H | Cl | 205-209 |
| **17** | 4-C₂H₅-Phenyl | H | Cl | 97- 98 |
| **18** | 3-CF₃-Phenyl | H | Cl | 74- 77 |
| **19** | 4-OCH₃-Phenyl | H | Cl | 153-155 |
| **20** | 4-F-Phenyl | Cl | Cl | 138-140 |
| **21** | 3Cl,4Cl-Phenyl | Cl | Cl | 162-165 |
| **22** | Cl | Cl | Phenyl | 170-173 |
| **23** | Cl | Cl | 3-Br-Phenyl | 196-200 |

### Beispiel 24

87,5 g 1-Cyano-3-phenyl-6-chlorpyridazinon-4 (0,378 Mol) wurden mit 136 g Natriumjodid in 700 ml Aceton gelöst und 88 Stunden auf Rückfluß erhitzt. Danach wurde das Lösungsmittel abgedampft und der Rückstand mit Wasser verrieben. Der dabei entstandene Feststoff wurde abfiltriert und getrocknet.
Dabei wurden 116 g 1-Cyano-3-phenyl-6-jodpyridazinon-4, das sind 95 % der Theorie, erhalten.
Nach Reinigung mittels Säulenchromatographie (SiO₂, CHCl₃) und nach folgender Umkristallisation aus Diisopropylether : Aceton 7 : 3 wurde ein Schmelzpunkt von 135 bis 137 °C gemessen.

### Beispiel 25

20,6 g 3-Phenyl-4-hydroxy-6-chlorpyridazin (0,1 Mol) wurden mit einer Lösung von 85,8 g Phosphoroxidbromid (0,3 Mol) in 150 ml Toluol bei Raumtemperatur unter Rühren versetzt, worauf 3 Stunden auf Rückfluß erhitzt wurde. Dabei entstand Bromwasserstoffgas, das abgezogen wurde. Nach beendeter Reaktion wurde das Reaktionsgemisch auf Eis gegossen, wobei ein teigiges Reaktionsprodukt erhalten wurde, das beim Anreiben in Petroläther erstarrte. Der Feststoff wurde abfiltiert, mit Wasser gewaschen und getrocknet.
Dabei wurden 27,1 g 3-Phenyl-4,6-dibrompyridazin, das sind 88 % der Theorie, mit einem Schmelzpunkt von 145-146 °C erhalten.

### Beispiel 26

47,0 g 3-Phenyl-4,6-dibrompyridazin (0,15 Mol) wurden in 300 ml Dioxan auf Rückfluß erhitzt und mit 17 g Natriumhydroxid (0,43 Mol), gelöst in 50 ml Wasser, innerhalb von 10 Minuten versetzt. Nach 4,5 Stunden auf Rückflußtemperatur wurde das Dioxan abgedampft und der feste Rückstand in heißem Wasser gelöst. Nach Abfiltieren eines teerigen Rückstandes mit Hilfe von Aktivkohle wurde Salzsäure bis zur deutlich sauren Reaktion zugegeben, wobei ein Feststoff ausfiel. Der Feststoff wurde abfiltiert, in Wasser aufgeschlämmt und durch Zugabe von wäßrigen Ammoniak unter Erhitzen gelöst. Durch erneutes Ansäuern mit Salzsäure wurde der Feststoff wieder ausgefällt, abfiltriert, mit Wasser gewaschen und getrocknet.
Dabei wurden 24,2 g 3-Phenyl-4-hydroxy-6-brom-pyridazin, das sind 64 % der Theorie, mit einem Schmelzpunkt von 210 bis 215 °C erhalten.

### Beispiel 27

96,1 g 3-(3'-Trifluormethylphenyl)-pyridazinon-6 wurden in 400 ml Phosphoroxidchlorid suspendiert und mit 12,4 g rotem Phosphor versetzt. In die Suspension wurde langsam Chlorgas eingeleitet, wobei die Temperatur anstieg, die dann durch Erwärmen der Reaktionsmischung von außen auf etwa 95 °C gehalten wurden. Nach 1,75 Stunden war die Reaktion beendet. Die Reaktionsmischung wurde auf Raumtemperatur abgekühlt und auf Eis gegossen, wobei die entstandene Säure durch gleichzeitige Zugabe von wäßrigem Ammoniak neutralisiert wurde. Der dabei anfallende Feststoff wurde abfiltriert, getrocknet und aus Hexan umkristallisiert.

Dabei wurden 84,4 g 3-(3'-Trifluormethylphenyl)-4,6-dichlorpyridazin, das sind 72 % der Theorie, mit einem Schmelzpunkt von 80 bis 81,5 °C erhalten.

### Beispiele 28 - 32

Auf die im Beispiel 16 beschriebene Art und Weise, unter Verwendung der entsprechenden Ausgangsstoffe wurden
28. 3-(4'-Bromphenyl)-4,6-dichlorpyridazin mit einem Schmelzpunkt von 170 bis 171 °C,
29. 3-(3',4'-Dichlorphenyl)-4,6-dichlorpyridazin mit einem Schmelzpunkt von 153 bis 154,5 °C,
30. 3-(2'-Fluorphenyl)-4,6-dichlorpyridazin mit einem Schmelzpunkt von 96 bis 98 °C,
31. 3-(4'-Cyanophenyl)-4,6-dichlorpyridazin mit einem Schmelzpunkt von 210 bis 222 °C und
32. 3-(4'-Äthylphenyl)-4,6-dichlorpyridazin mit einem Schmelzpunkt von 35 bis 39 °C
erhalten.

### Beispiel 33

70 g 3-Phenylpyridazinon-6 (0,41 Mol) wurden mit 10 g rotem Phosphor in 300 ml Phosphoroxidchlorid suspendiert. In diese Suspension wurde Chlorgas unter Rühren eingeleitet, wobei die Reaktionstemperatur, die dabei zuerst anstieg, erst durch Kühlen, dann durch Erwärmen der Reaktionsmischung auf Temperaturen von 70 bis 75 °C gehalten wurde. Nach 3 Stunden wurde die Reaktionsmischung auf Raumtemperatur abkühlen gelassen und auf Eis gegossen, wobei die entstandene Säure durch Zugabe von wäßrigem Ammoniak neutralisiert wurde. Der erhaltene Feststoff wurde abfiltriert, mit Wasser gewaschen und aus Äthanol zwei Mal umkristallisiert.
Dabei wurden 88,9 g 3,4,5-Trichlor-6-phenyl-pyridazin, das sind 84 % der Theorie, mit einem Schmelzpunkt von 120 bis 121,5 °C erhalten .

### Beispiele 34 - 36

Auf die in Beispiel 22 beschriebene Art und Weise, unter Verwendung der entsprechenden Ausgangsstoffe wurden
34. 3,4,5-Trichlor-6-(4'-bromphenyl)-pyridazin mit einem Schmelzpunkt von 164 bis 167 °C,
35. 3,4,5-Trichlor-6-(4'-chlorphenyl)-pyridazin mit einem Schmelzpunkt von 155 bis 159 °C und
36. 3,4,5-Trichlor-6-(4'-fluorphenyl)-pyridazin mit einem Schmelzpunkt von 153 bis 155 °C erhalten.

### Beispiel 37

75 g 3,4,5-Trichlor-6-(4'-chlorphenyl)-pyridazin (0,255 Mol) wurden bei Siedehitze in 300 ml Dioxan gelöst und mit 100 ml Wasser versetzt. Dieser Losung wurde innerhalb von 30 Minuten eine Lösung von 20,4 g Natriumhydroxid in 150 ml Wasser zugetropft. Das Lösungsmittel wurde abgedampft und der Rückstand in heißem Wasser gelöst. Durch Zugabe von wäßriger Salzsäure wurde ein Feststoff ausgefällt, der abfiltiert und zur Reinigung durch Lösen in einer verdünnten, wäßrigen Ammoniaklösung und anschließende Zugabe von Salzsäure umgefällt wurde. Der Feststoff wurde abfiltriert und aus einem Äthanol/Dimethylsulfoxidgemisch umkristallisiert, wobei 36,6 g 3,5-Dichlor-4-hydroxy-6-(4'-chlorphenyl)-pyridazin, das sind 52 % der Theorie mit einem Schmelzpunkt von 272 bis 278 °C (Zersetzung) erhalten wurde.

### Beispiele 38 - 40

Auf die im Beispiel 26 beschriebene Art und Weise, unter Verwendung der entsprechenden Ausgangsstoffe wurden
38. 3,5-Dichlor-4-hydroxy-6-phenyl-pyridazin mit einem Schmelzpunkt von 190 bis 193 °C,
39. 3,5-Dichlor-4-hydroxy-6-(4'-fluorphenyl)-pyridazin mit einem Schmelzpunkt von 266 bis 270 °C und
40. 3,5-Dichlor-4-hydroxy-6-(4'-bromphenyl)-pyridazin mit einem Schmelzpunkt von 276 bis 281 °C hergestellt.

### Beispiel 41

45 g 3-Phenyl-4,6-dichlor-pyridazin (0,2 Mol) wurden in 600 ml Methanol gelöst und mit 108 g einer 30%igen Lösung von Natriummethylat in Methanol (0,6 Mol) versetzt, worauf die Reaktionsmischung 7 Stunden auf Rückfluß erhitzt wurde. Nach Abkühlen der Reaktionsmischung wurde vom ausgefallenen Natriumchlorid abfiltriert und die organische Phase abgedampft. Der Rückstand wurde in Methylenchlorid/Wasser aufgenommen, die organische Phase mit Wasser ausgeschüttelt, über Natriumsulfat getrocknet und abgedampft. Der Rückstand wurde aus Cyclohexan umkristallisiert. Dabei wurden 39,9 g 3-Phenyl-4,6-dimethoxy-pyridazin, das sind 92 % der Theorie, mit einem Schmelzpunkt von 53 bis 54 °C erhalten.

### Beispiel 42

Auf die in Beispiel 41 beschriebene Art und Weise, aber unter Verwendung von Natriumbutylat anstatt Natriummethylat, wurde 4,6-Dibutoxy-3-phenylpyridazin in Form eines hellen, chlorfreien Öles erhalten.

### Beispiel 43

39,9 g 3-Phenyl-4,6-dimethoxy-pyridazin (0,18 Mol) wurden in 500 ml Dimethylformamid gelöst und bei Raumtemperatur mit 4,9 g einer 80%igen Suspension von Natriumhydrid in Mineralöl versetzt. Anschließend wurde dem Reaktionsgemisch 18,4 g Butanthiol (0,2 Mol) zugetropft, wobei leichte Erwärmung eintrat. Nach einigen Stunden Rühren bei Raumtempertur wurde 5 Stunden auf 100 °C erhitzt, worauf das Lösungsmittel abgedampft wurde. Der Rückstand wurde in Wasser/Cyclohexan aufgenommen, die wäßrige Phase mit Cyclohexan ausgeschüttelt und durch Zugabe von Essigsäure angesäuert, wobei ein Feststoff ausfiel, der abfiltriert, mit Wasser gewaschen und getrocknet wurde. Dabei wurden 32,5 g 3-Phenyl-4-hydroxy-6-methoxy-pyridazin, das sind 87 % der Theorie erhalten, das nach dem Umkristallisieren aus einem Gemisch von Methanol : Dimethylformamid = 1 : 1 einem Schmelzpunkt von 204 bis 209 °C aufwies.

### Beispiel 44

Auf die in Beispiel 41 beschriebene Art und Weise wurde ausgehend von 3-Phenyl-4,6-dibutoxy-pyridazin 3-Phenyl-4-hydroxy-6-butoxy-pyridazin mit einem Schmelzpunkt von 217 bis 220 °C erhalten.

### Beispiel 45

0,5 Teile der Verbindung 1 wurden mit 5 Teilen Na-N-Oleoyl-N-methyltaurid, 3 Teilen Na-Diisobutylnaphthalinsulfonat, 10 Teilen Ca-Ligninsulfonat und 81,5 Teilen Kaolin innig vermengt und anschließend in einer Planetenkugelmühle 1 Stunde lang gemahlen. Dabei wurde ein benetzbares Pulver, das sich zur Herstellung einer herbiziden Spritzbrühe eignet, erhalten.

### Beispiel 46

10 Teile der Verbindung 1 wurden in 30 Teilen Xylol und 40 Teilen N-Methylpyrrolidon gelöst und mit 10 Teilen eines Emulgator-Gemisches, bestehend aus Ca-Dodecylbenzolsulfonat und äthoxylierter Tallölfettsäure, versetzt.
Dabei wurde ein emulgierbares Konzentrat, das sich zur Herstellung einer herbiziden Spritzbrühe eignet, erhalten.

### Beispiel 47

25 Teile der Verbindung 1 wurden mit 5 Teilen Na-N-Oleoyl-N-methyltaurid, 3 Teilen Na-Diisobutylnaphthalinsulfonat, 10 Teilen Ca-Ligninsulfonat, 25 Teilen Attaclay^{R} und 32 Teilen Kaolin innig vermengt und anschließend in einer Planetenkugelmühle eine Stunde lang gemahlen.
Dabei wurde ein benetzbares Pulver, das sich zur Herstellung einer herbiziden Spritzbrühe eignet, erhalten.

### Beispiel 48

60 Teile der Verbindung 1 wurden mit 5 Teilen Na-N-Oleoyl-N-methyltaurid, 3 Teilen Na-Laurylsulfat, 10 Teilen Ca-Ligninsulfonat und 22 Teilen Attaclay^{R} innig vermengt und anschließend in einer Planetenkugelmühle eine Stunde lang gemahlen.

Dabei wurde ein benetzbares Pulver, das sich zur Herstellung einer herbiziden Spritzbrühe eignet, erhalten.

### Beispiel 49

90 Teile der Verbindung 1 wurden mit 4 Teilen Na-N-Oleoyl-N-methyltaurid, 2 Teilen Na-Diisobutylnaphthalinsulfonat und 4 Teilen gefällter Kieselsäure innig vermengt und anschließend in einer Planetenkugelmühle eine Stunde lang gemahlen.
Dabei wurde ein benetzbares Pulver, das sich zur Herstellung einer herbiziden Spritzbrühe eignet, erhalten.

Zur Überprüfung der Wirksamkeit der neuen N-Cyanopyridazinone wurde eine bestimmte Wirkstoffmenge im Nachlaufverfahren (3 bis 6-Blattstadium) auf die Testpflanzen appliziert. Die Bonitur erfolgte 2 bis 3 Mal. Die dargestellten Werte sind Duchschnittswerte aller Bonituren. Die Auswertung erfolgte nach dem Boniturschema der EWRC-Scala 1-9 der Tabelle 2.

**Tabelle 2**

| **Note** | **Herbizide Wirkung** | **in %** |
|---|---|---|
| 1 | ausgezeichnet | 100 |
| 2 | sehr gut | > 97,5 |
| 3 | gut | > 95,0 |
| 4 | befriedigend | > 90,0 |
| 5 | noch ausreichend | > 85,0 |
| 6 | nicht ausreichend | < 85,0 |
| 7 | gering | < 75,0 |
| 8 | sehr gering | < 65,0 |
| 9 | keine Wirkung | < 32,5 |

Als Vergleichssubstanz wurde das bekannte Herbizid Lentagran der Chemie Linz AG (6-Chloro-3-phenylpyridazin-4-yl-S-octylthiocarbonat) herangezogen. Die Dosierung ist in Gramm Wirkstoff pro Hektar angegeben. Die Präparate wurden teilweise mit Nopon 11E ausgebracht. NOPON 11E ist ein käufliches Präparat der Firma Sun Oil und besteht zu 99 % aus Paraffinöl und zu einem Prozent aus Emulgatoren.

### Bespiel A

| **Präparat** | **Dosierung** | **herbizide Wirkung** | | |
|---|---|---|---|---|
| | | **STEME** | **LAPCO** | **ANTAR** |
| Lentagran | 250 | 8,0 | 5,5 | 7,0 |
| Verbindung 1 | 250 | 6,5 | 2,0 | 1,0 |

### Beispiel B

Die Wirkstoffe wurden jeweils mit 5,0 Liter NOPON 11E pro Hektar ausgebracht.

| **Präparat** | **Dosierung** | **herbizide Wirkung in % ECHCG** |
|---|---|---|
| Lentagran | 600 | 78 |
| Lentagran | 450 | 38 |
| Verbindung 1 | 367 | 78 |
| Verbindung 1 | 275 | 56 |
| Verbindung 2 | 437 | 75 |
| Verbindung 2 | 327 | 58 |
| Verbindung 15 | 406 | 69 |
| Verbindung 15 | 304 | 66 |

### Beispiel C

Die Wirkstoffe wurden jeweils mit 2,5 Liter NOPON 11E pro Hektar ausgebracht.

| **Präparat** | **Dosierung** | **herbizide Wirkung** | |
|---|---|---|---|
| | | **CHEAL** | **AMARE** |
| Lentagran | 300 | 5,0 | 2,7 |
| Lentagran | 225 | 6,3 | 3,3 |
| Verbindung 1 | 183 | 4,0 | 2,7 |
| Verbindung 1 | 138 | 6,3 | 2,7 |
| Verbindung 2 | 218,4 | 2,3 | 2,0 |
| Verbindung 2 | 163,5 | 3,3 | 2,3 |
| Verbindung 11 | 246 | 1,3 | 2,3 |
| Verbindung 11 | 184 | 2,0 | 3,7 |
| Verbindung 13 | 238 | 1,3 | 2,0 |
| Verbindung 13 | 178 | 2,3 | 2,3 |
| Verbindung 15 | 203 | 1,3 | 2,0 |
| Verbindung 15 | 152 | 1,3 | 1,7 |

## Patentansprüche

1. N-Cyanopyridazinone der allgemeinen Formel 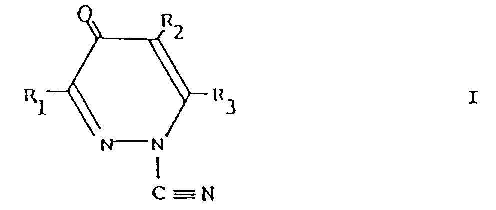 in der R₁, R₂ und R₃ unabhängig voneinander Wasserstoff, Halogen-, Amino-, Nitro-, Cyano-, Alkyl-, Aryl-, Aralkyl-, Alkoxy- oder Aryloxygruppen bedeuten.

2. N-Cyanopyridazinone nach Anspruch 1, dadurch gekennzeichnet, daß R₁, R₂ und R₃ unabhängig voneinander Wasserstoff, eine Halogen-, Alkoxy- oder eine Arylgruppe bedeuten.

3. N-Cyanopyridazinone nach einem der Ansprüche 1 oder 2, dadurch gekennzeichnet, daß R₁ eine Halogen- oder Arylgruppe bedeutet.

4. N-Cyanopyridazinone nach einem der Ansprüche 1 bis 3, dadurch gekennzeichnet, daß R₁ eine Halogen- oder Arylgruppe, R₂ Wasserstoff oder eine Halogengruppe und R₃ eine Alkoxy-, Halogen- oder Arylgruppe bedeuten.

5. N-Cyanopyridazinone nach einem der Ansprüche 1 bis 4, dadurch gekennzeichnet, daß die Arylgruppe eine unsubstituierte oder ein- oder mehrfach durch Halogen-, Cyano-, Nitro- oder Alkylgruppen substituierte Phenylgruppe ist.

6. Verfahren zur Herstellung von N-Cyanopyridazinonen der allgemeinen Formel 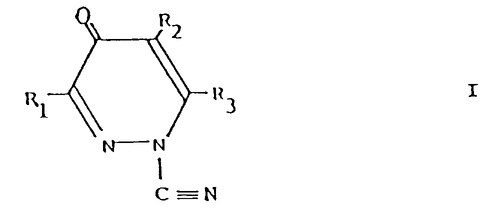 in der R₁, R₂ und R₃ unabhängig voneinander Wasserstoff, Halogen-, Amino-, Nitro-, Cyano-, Alkyl-, Aryl-, Aralkyl-, Alkoxy- oder Aryloxygruppen bedeuten, das dadurch gekennzeichnet ist, daß ein Hydroxypyridazin der allgemeinen Formel 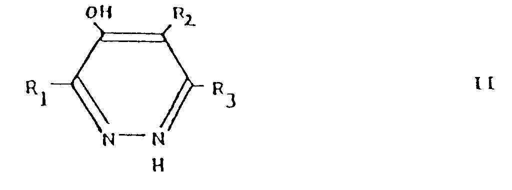 in der R₁, R₂ und R₃ die obgenannte Bedeutung haben, in einem unter den Reaktionsbedingungen inerten Verdünnungsmittel gelöst oder suspendiert, mit einer Base und mit einem Halogencyan versetzt und reagieren gelassen wird, worauf das gebildete Cyanopyridazinon der allgemeinen Formel I aus dem Reaktionsgemisch isoliert wird.

7. Verfahren nach Anspruch 6, dadurch gekennzeichnet, daß als Base ein Amin und als Halogencyan Chlorcyan oder Bromcyan eingesetzt werden.

8. Herbizides Mittel, dadurch gekennzeichnet, daß es mindestens ein N-Cyanopyridazinon der allgemeinen Formel 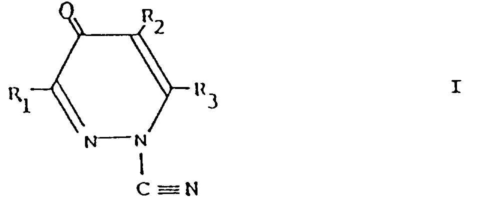 in der R₁, R₂ und R₃ unabhängig voneinander Wasserstoff, Halogen, Amino-, Nitro-, Cyano-, Alkyl-, Aryl-, Aralkyl-, Alkoxy- oder Aryloxygruppen bedeuten, neben Hilfs- und/oder Trägerstoffen enthält.

9. Verfahren zur Herstellung eines herbiziden Mittel, dadurch gekennzeichnet, daß mindestens ein N-Cyanopyridazinon der allgemeinen Formel I nach Anspruch 1 mit Hilfs- und/oder Trägerstoffen vermischt wird.

10. Verfahren zur Bekämpfung von Unkräutern, dadurch gekennzeichnet, daß ein N-Cyanopyridazinon der Formel I nach Anspruch 1 oder ein herbizides Mittel nach Anspruch 8 auf Unkräuter oder deren Lebensraum einwirken gelassen wird.
